# EUROPEAN PATENT SPECIFICATION

(11) **EP 2 276 472 B1**
(45) Date of publication and mention of the grant of the patent: **21.11.2018**
(21) Application number: 09732469.3
(22) Date of filing: 15.04.2009
(51) Int. Cl.: A61K 9/16, A61K 9/20, A61K 9/50

(54) **COMPOSITIONS COMPRISING WEAKLY BASIC DRUGS AND CONTROLLED-RELEASE DOSAGE FORMS**
ZUSAMMENSETZUNGEN MIT SCHWACH BASISCHEN ARZNEIMITTELN UND DOSIERFORMEN MIT KONTROLLIERTER FREISETZUNG
COMPOSITIONS COMPRENANT DES MÉDICAMENTS FAIBLEMENT BASIQUES ET FORMES POSOLOGIQUES À LIBÉRATION CONTRÔLÉE

(30) Priority: 15.04.2008 US 45170 P
(43) Date of publication of application: 26.01.2011
(73) Proprietor: Adare Pharmaceuticals, Inc., Lawrenceville, NJ 08648 (US)
(72) Inventor: VENKATESH, Gopi, Vandalia OH 45377 (US); LAI, Jin-wang, Springboro OH 45066 (US); STEVENS, Philip, J., Englewood OH 45322 (US)
(74) Representative: Cooley (UK) LLP
(86) International application number: PCT/US2009/040608
(87) International publication number: WO 2009/129282

(56) References cited:
- WO-A2-2007/122478
- US-A- 3 341 416
- US-A1- 2005 214 372
- US-A1- 2005 232 988
- US-A1- 2005 287 211
- US-A1- 2006 240 100
- US-A1- 2006 246 134
- US-A1- 2007 190 145
- US-A1- 2007 196 491
- Evonik industries AG: "Eudragit L 100-55", , December 2012 (2012-12), XP002694459, Retrieved from the Internet: URL:http://eudragit.evonik.com/product/eud ragit/Documents/evonik-specification-eudra git-l-100-55.pdf [retrieved on 2013-03-26]

## Description

### BACKGROUND OF THE INVENTION

Many therapeutic agents are most effective when made available at constant rates at or near the absorption sites. The absorption of therapeutic agents thus made available generally results in desired plasma concentrations leading to maximum efficacy and minimum toxic side effects. Much effort has been devoted to developing sophisticated drug delivery systems such as osmotic devices for oral application. However, there are instances where simple drug delivery systems such matrix tablets comprising dissolution rate-controlling polymers, or monolithic or multiparticulate systems coated with functional polymers fail to provide target pharmacokinetic (PK) profiles suitable for once- or twice-daily dosing regimens.

For adequate absorption of a drug from the gastrointestinal tract, the drug should be released from the dosage form and be available in solution form at or near the absorption site. The rate at which the drug goes into solution and releases from a dosage form is important to the kinetics of drug absorption. The dosage form and hence the active ingredient are subjected to varying pHs during the transit, varying from about pH 1.2 (stomach during fasting) to about 7.0 (bile or intestinal). Moreover, transit time of a dosage form in individual parts of the digestive tract may vary significantly depending on the size of the dosage form and the local conditions within the digestive tract. Other factors that influence drug absorption include physicochemical properties of the drug substance itself such as pKa, solubility, crystalline energy, and specific surface area. The prevailing local conditions within the digestive tract that play an important role include properties of luminal contents (pH, surface tension, volume, agitation and buffer capacity) and changes following the ingestion of food. Consequently, it is often difficult to achieve drug release at constant rates, especially in case of very soluble or freely soluble weakly basic drugs, that are rapidly released under acidic pH conditions, thereby resulting in dose dumping. Functional polymer membranes comprising suitable combinations of synthetic polymers, such as water-soluble polymers (e.g., povidone), water-insoluble polymers (e.g., ethylcellulose), or enterosoluble polymers (e.g., gastric-resistant hypromellose phthalate), have been applied to tablets or pellet cores comprising the drug to achieve sustained release profiles with limited success.

Orally disintegrating dosage forms have grown steadily in popularity as more convenient and potentially safer alternatives to conventional tablets and capsules. These rapidly disintegrating dosage forms disintegrate in the oral cavity, and they are easily swallowed without water. They are a boon to the 50% of the population who have difficulty swallowing conventional tablets and capsules (common among geriatric and pediatric patients); people who do not have ready access to water (e.g., bed-ridden or immobile patients, or active people often away from home); and caregivers whose patients are reluctant to take their medications. Orally disintegrating dosage forms help to improve patient compliance with oral dosage regimens because they are easy to administer, convenient to take discreetly anywhere, and difficult to discard once administered. However, these dosage forms are not only required to rapidly disintegrate on contact with the saliva in the oral cavity but also must have acceptable organoleptic properties (i.e., be palatable) and pharmacokinetic properties (i.e., rate and duration of drug release) appropriate for the particular drug and the condition treated. These properties are often mutually antagonistic. Thus, the development of orally disintegrating tablets (ODTs) containing weakly basic drugs that are freely soluble in the physiological pH range of 1.2 to 6.8 for once- or twice-daily dosing regimen is particularly challenging.

Weakly basic drugs are rapidly released under acidic conditions and hence often fail to provide target PK profiles suitable for a once- or twice-daily dosing regimen. Furthermore, basic drugs are difficult to work with, if high doses are required to be therapeutically effective, because the solubility decreases by about 1-2 orders of magnitude on transit from the stomach to the colon. If extremely thick polymer coatings are applied with the intention of sustaining the drug release in the lower pH region i.e., pH 1.2 - 6.8, the drug is released by diffusion through the coating membrane at such a slow rate to be of practical utility.

Co-pending US Patent Applications Ser. No. 11/668,167 (published as US 2007/0190145) and US Patent Application No. 11/668,408 (published as US 2007/0196491), both filed January 29, 2007 disclose pharmaceutical compositions comprising separate layers of weakly basic drugs and an organic acid.
US Publication Nos. US 2005/287211 and US 2005/214372 and International Publication No. WO 2007/122478 all concern pharmaceutical compositions comprising an acid labile drug covered by an outer enteric layer.

### SUMMARY OF THE INVENTION

In a first aspect, the invention relates to a pharmaceutical composition comprising a plurality of controlled-release particles, wherein at least one population of said particles comprises: (a) a core comprising a weakly basic drug, or a pharmaceutically acceptable salt, solvate, and/or ester thereof; (b) an alkaline-buffer layer deposited over the core, comprising an alkaline buffer, wherein the alkaline-buffer layer may or may not be in physical contact with the core; and (c) a controlled-release coating deposited over the alkaline-buffer layer, wherein the controlled-release coating may or may not be in physical contact with the alkaline-buffer layer; wherein the controlled-release coating comprises a water-insoluble polymer in the absence of a water-soluble or enteric polymer, and sustains drug release over from 8 hours to 20 hours, wherein the release is determined in a two-stage dissolution method (700 mL of 0.1N hydrochloric acid for the first 2 hours and thereafter in 900 mL at pH 6.8 obtained by adding 200 mL of a pH modifier).

In one embodiment, the present invention relates to a pharmaceutical composition comprising a plurality of controlled-release particles, wherein each particle comprises a core comprising a weakly basic drug containing at least one nitrogen-containing moiety with a pKa of from about 5 to about 14, a solubility of at least 200 mg/mL in a room-temperature aqueous solution at about pH 1.2 - 6.8, and a solubility of not more than about 10 mg/mL at pH 8 or higher; an alkaline buffer layer disposed over the drug core; and a controlled-release coating disposed over the alkaline buffer layer, wherein the controlled-release coating comprises a water-insoluble polymer.

In a second aspect, the present invention relates to a method of preparing a pharmaceutical composition comprising a plurality of controlled-release particles, comprising: (a) preparing a core comprising a weakly basic drug; (b) coating the drug-containing core of step (a) with a layer comprising an alkaline buffer; and (c) coating the alkaline-buffer layered core of step (b) with a controlled-release coating comprising a water-insoluble polymer in the absence of a water-soluble or enteric polymer, which sustains drug release over from 8 hours to 20 hours, wherein the release is determined in a two-stage dissolution method (700 mL of 0.1N hydrochloric acid for the first 2 hours and thereafter in 900 mL at pH 6.8 obtained by adding 200 mL of a pH modifier).

In another embodiment, the present invention relates to a pharmaceutical dosage form comprising a composition of the first aspect. Each particle in the composition comprises a core comprising a weakly basic drug; an alkaline buffer layer disposed over the core; and a controlled-release coating disposed over the alkaline buffer layer, wherein the controlled-release coating comprises a water-insoluble polymer.

In yet another embodiment, the present invention relates to a pharmaceutical dosage form comprising a composition of the first aspect in which there are at least two populations of drug particles. One population of drug particles comprises cores comprising a weakly basic drug in accordance with the first aspect while the second population of drug particles comprises cores comprising a weakly basic drug, an alkaline buffer layer disposed over the drug core; and a controlled-release coating disposed over the alkaline buffer layer, wherein the controlled-release coating comprises a water-insoluble polymer alone or in combination with an enteric polymer.

In yet another embodiment, the present invention relates to a pharmaceutical dosage form comprising a composition of the first aspect in which there are at least two populations of drug particles. One population of drug particles comprises cores comprising a weakly basic drug in accordance with the first aspect while the second population of drug particles comprises cores comprising a weakly basic drug, an alkaline buffer layer disposed over the drug core; and a controlled-release coating disposed over the alkaline buffer layer, wherein the controlled-release coating comprises a water-insoluble polymer alone or in combination with a water-soluble polymer.

The present disclosure also relates to a method of preparing the pharmaceutical dosage form. In one embodiment, the pharmaceutical dosage form is prepared by mixing the microparticles described herein with rapidly disintegrating granules comprising a saccharide and/or a sugar alcohol in combination with a disintegrant to form a compression blend, and compressing the blend into a tablet. In another embodiment, the pharmaceutical dosage form is prepared by filling the microparticles described herein into a capsule.

### BRIEF DESCRIPTION OF THE DRAWINGS

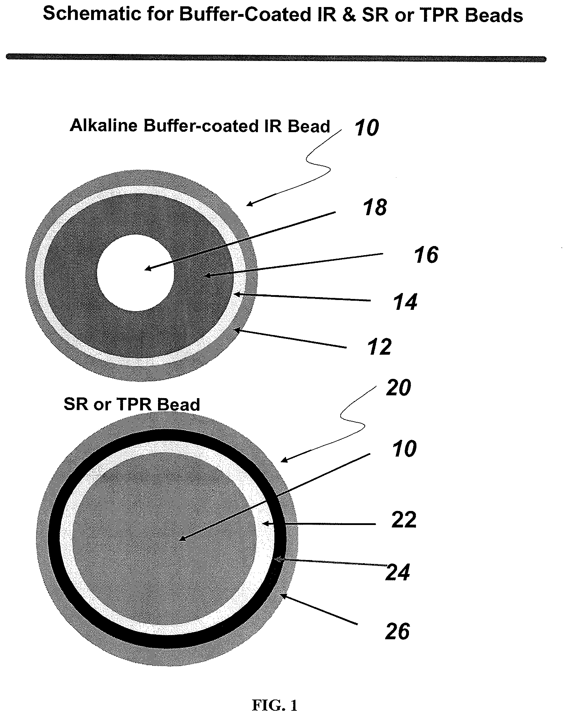

FIG. 1 illustrates cross-sections of an alkaline buffer-coated IR bead (upper drawing) and a SR or TPR bead comprising an alkaline buffer coated IR bead comprising a weakly basic drug in accordance with particular embodiments of the invention (lower drawing). In Fig. 1 (top schematic), an alkaline buffer coated IR bead 10 comprises an alkaline buffer layer 12 disposed over a protective sealant layer 14, which is disposed over a weakly basic drug layer 16 disposed over an inert core 18 comprising sugar, lactose sphere, microcrystalline cellulose, mannitol-microcrystalline cellulose, or silicon dioxide. In the same figure (bottom schematic), the SR or TPR bead 20 comprises a compressible coating layer 26 disposed over an controlled-release coating (SR or TPR layer) 24, which is disposed over a sealant layer 22, which is disposed over an alkaline buffer coated IR bead 10.

### DETAILED DESCRIPTION OF THE INVENTION

The citation of any document is not to be construed as an admission that it is prior art with respect to the present invention.

The terms "drug", "active" or "active pharmaceutical ingredient" as used herein include a pharmaceutically acceptable and therapeutically effective compound, pharmaceutically acceptable salts, stereoisomers and mixtures of stereoisomers, solvates (including hydrates), polymorphs, and/or esters thereof. When referring to a drug in the descriptions of the various embodiments of the invention, the reference encompasses the base drug, pharmaceutically acceptable salts, stereoisomers and mixtures of stereoisomers, solvates (including hydrates), polymorphs, and/or esters thereof.

The terms "orally disintegrating tablet" or "ODT" refers to a tablet which disintegrates rapidly in the oral cavity of a patient after administration, without e.g. the need for chewing. The rate of disintegration can vary, but is faster than the rate of disintegration of conventional solid dosage forms (i.e., tablets or capsules) which are intended to be swallowed immediately after administration, or of chewable solid dosage forms.

The term "about", as used herein to refer to a numerical quantity, includes "exactly". For example, "about 60 second" includes 60 seconds, exactly, as well as values close to 60 seconds (e.g., 50 seconds, 55 seconds, 59 seconds, 61 seconds, 65 seconds, 70 seconds, etc.).

The term "weakly basic drug" encompasses drugs containing one or more nitrogen moieties with a pKa in the range of from about 5 to about 14 that are very soluble to freely soluble under acidic and neutral pH conditions (i.e. at a pH from about 1.2 up to a pH of about 6.8), but poorly soluble above pH 6.8. The terms referring to solubility (e.g., "very soluble," "freely soluble," "poorly soluble," etc.) have the same meaning as defined in the U.S. Pharmacopeia (Vol. 26, NF 21, 2003), with the understanding that the solubility limits provided represent approximate limits. For example, "very soluble" means having a solubility of not less than about 1g solute per 1 mL of water or aqueous solution at room temperature at a specified pH; "freely soluble" means having a solubility of about 100 to about 1000 mg solute per 1 mL of water or aqueous solution at room temperature at a specified pH; "poorly soluble" means having a solubility of less than about 100 mg solute per 1 mL of water at room temperature.

As used herein, the term "controlled-release" coating encompasses coatings that delay release, extend release, sustain release, prevent release, and/or otherwise prolong the release of a drug from a particle coated with a controlled-release coating. The term "controlled-release" encompasses "sustained-release" and "timed, pulsatile release." A controlled-release coating may also be referred to herein as a "lag-time" coating.

As used herein, the term "immediate-release core" refers to a core containing a drug, optionally layered with a sealant layer, but not coated with a controlled-release coating. An "immediate-release core" can include drug crystals (or amorphous particles), granulates of the drug with one or more excipients, or an inert core (e.g., a sugar sphere) layered with a drug (and an optional binder), a protective sealant coating and an optional alkaline buffer layer. Immediate-release cores" have immediate release properties as described herein. Extended release particles (e.g., SR particles, TPR particles, etc.) can be prepared by coating immediate-release cores with an extended release coating.

As used herein, the term "immediate release" or IR refers to release of greater than or equal to about 50% (especially if taste-masked for incorporation into an orally disintegrating tablet dosage form), preferably greater than about 75%, more preferably greater than about 90%, and in accordance with certain embodiments greater than about 95% of the active within about 2 hours, more particularly within about one hour following administration of the dosage form.

The term "TPR particle" or "TPR bead" refers to a drug-containing particle, e.g., a drug-layered bead, drug-containing granulate, or drug particle, coated with a TPR ("timed pulsatile release") coating. The TPR coating provides an immediate release pulse of the drug, or a sustained drug-release profile after a pre-determined lag time. The term "lag-time" refers to a time period after oral administration of the drug-containing particle or after exposure to the 2-stage dissolution media or simulated body fluid(s), wherein less than about 10% of the drug is released from the drug-containing particle. In one embodiment, the term "lag-time" refers to a time period wherein substantially none of the drug is released from the particle or after exposure to the 2-stage dissolution media or simulated body fluid(s). In some embodiments, a lag-time of from at least about 1 to 10 hours is achieved by coating the particle with, e.g. a combination of at least one water-insoluble polymer and at least one enteric polymer (e.g., a combination of ethylcellulose and hypromellose phthalate). In one embodiment, the lag time ranges from about 2 to about 10 hours. The TPR layer can optionally contain a plasticizer.

The term "sustained-release coating" or "SR coating" refers to a coating providing sustained release properties, e.g. a coating which slows the release of the drug from the drug- and "containing particle but does not provide an appreciable "lag-time." In one disclosed example, and SR coating comprises a water-insoluble polymer and optionally a water-soluble polymer.

The term "substantially disintegrates" means a level of disintegration amounting to disintegration of at least about 50%, at least about 60%, at least about 70%, at least about 80%, at least about 90%, or about 100% disintegration of the ODT composition.

The term "substantially masks the taste" in reference to the taste-masking layer of the IR particles (when present) refers to the ability of the taste masking layer to substantially prevent release of a bitter tasting drug in the oral cavity of a patient. A taste-masking layer which "substantially masks" the taste of the drug typically releases less than about 10% of the drug in the oral cavity of the patient, in other embodiments, less than about 5%, less than about 1%, less than about 0.5%, less than about 0.1%, less than about 0.05%, less than about 0.03%, less than about 0.01% of the drug. The taste-masking properties of the taste-masking layer of the compositions of the present invention can be measured in vivo (e.g., using conventional organoleptic testing methods known in the art) or in vitro (e.g., using dissolution tests as described herein). The skilled artisan will recognize that the amount of drug release associated with a taste-masking layer that "substantially masks" the taste of a drug is not limited to the ranges expressly disclosed herein, and can vary depending on other factors such as the perceived the bitterness of the drug and, e.g. the presence of flavoring agents in the composition.

The terms "plasma concentration-time profile," "Cₘₐₓ," "AUC," "Tₘₐₓ," and "elimination half life" have their generally accepted meanings as defined in the FDA Guidance for Industry: Bioavailability and Bioequivalence Studies for Orally Administered Drug Products (March 2003).

Unless stated otherwise, the amount of the various coatings or layers described herein (the "coating weight") is expressed as the percentage weight gain of the particles or beads provided by the dried coating, relative to the initial weight of the particles or beads prior to coating. Thus, a 10% coating weight refers to a dried coating which increases the weight of a particle by 10%. Unless stated otherwise, ratios are calculated by weight.

In one embodiment, the present invention relates to a pharmaceutical composition comprising a plurality of controlled-release particles, wherein each particle comprises a core comprising a weakly basic drug; an alkaline buffer layer disposed over the core; and a controlled-release coating disposed over the alkaline buffer layer. In the embodiments, the controlled-release coating comprises a water-insoluble polymer. In accordance with certain embodiments of the present invention, the pharmaceutical composition encompasses any weakly basic drug having at least one nitrogen-containing moiety, a pKa of from about 5 to about 14, and a solubility of at least about 200 mg/mL in a room-temperature aqueous solution at about pH 1.2 - 6.8, and a solubility of less than about 10 mg/mL at pH 8 or higher. Without being bound by the theory of drug release controlling mechanism, the alkaline buffer layer disposed over the weakly basic drug-containing core creates an alkaline pH micro environment at the drug interface wherein the drug is at best poorly soluble even when the exterior of the controlled-release coated bead is acidic wherein the drug is freely soluble, thereby avoiding dose dumping upon oral administration.

In some embodiments, the weakly basic drugs of the present invention can be selected from the following non-limiting examples of drug classes: analgesics, anticonvulsants, antidiabetic agents, anti-infective agents, antineoplastics, anti-Parkinsonian agents, antirheumatic agents, cardiovascular agents, central nervous system (CNS) stimulants, dopamine receptor agonists, anti-emetics, gastrointestinal agents, psychotherapeutic agents (e.g., antipsychotics), opioid agonists, opioid antagonists, anti-epileptic drugs, histamine H₂ antagonists, anti-asthmatic agents, and skeletal muscle relaxants.

Examples of weakly basic drugs include, but are not limited to, butyrophenone derivatives containing a nitrogen moiety, phenylamino imidazoline (e.g., clonidine, an antihypertensive agent), dihydroxyphenyl isopropylamino ethane (e.g., fenoterol, a broncholytic agent), phenoxy butylamino propanol (e.g., β-adrenolytic bunitrolol), phenoxy amino propane (e.g., antiarrhythmic mexiletine), amino ethyl oxazolo azepine (antihypertensive and anti-anginal agent) or a pharmaceutically acceptable salt, solvate, ester, polymorph, or mixture thereof. In some embodiments, the weakly basic drug has an elimination half-life of from about 2 hours to about 7 hrs.

The term "disposed over" means that a second material is deposited over a first material, wherein the second material may or may not be in physical contact with the first material. Thus it is possible, but not necessary, that an intervening material lies between the first and second materials.

The alkaline buffer layer is believed to create an alkaline microenvironment at the drug interface inside the controlled-release particle. Because the weakly basic drug has a lower solubility in this microenvironment, the alkaline buffer layer effectively delays release of the drug under the acidic to neutral pH conditions of the gastrointestinal tract, conditions under which the drug would otherwise dissolve rapidly. By incorporating an alkaline buffer layer into the compositions of the present invention, it is possible to achieve pharmacokinetic profiles suitable for a once- or twice-daily dosing regimen. Non-limiting examples of alkaline buffers suitable for the compositions of the present invention include sodium hydroxide, monosodium dihydrogen phosphate, disodium hydrogen phosphate, trisodium phosphate, sodium acetate, sodium carbonate or bicarbonate, monopotassium dihydrogen phosphate, dipotassium hydrogen phosphate, tripotassium phosphate, potassium acetate, potassium carbonate or bicarbonate, magnesium phosphate, magnesium acetate, magnesium carbonate, magnesium oxide, magnesium hydroxide, sodium silicate, calcium silicate, complex magnesium aluminum metasilicate, and mixtures thereof. The alkaline buffer layer optionally contains a polymeric binder. The polymeric binder can be selected from the group consisting of hydroxypropylcellulose, povidone, methylcellulose, hydroxypropyl methylcellulose, carboxyalkylcellulose, polyethylene oxide, and a polysaccharide.

Within the controlled-release particle, the alkaline buffer layer is disposed on the sealant layer, which in turn is disposed on the core comprising a weakly basic drug. In one embodiment, the alkaline buffer layer can include a polymeric binder, if necessary. Non-limiting examples of suitable polymeric binders include hydroxypropylcellulose, povidone, methylcellulose, hydroxypropyl methylcellulose, carboxyalkylcellulose, polyethylene oxide, starch, and polysaccharide. In some embodiments the ratio of the alkaline buffer to the weakly basic drug ranges from about 5:1 to about 1:5, including from about 3:1 to about 1:3.

The compositions of the present invention can, in some embodiments, comprise a sealant layer disposed on the drug-containing core, underlying the alkaline buffer layer. This protective sealant layer separates the drug-containing core and the alkaline buffer layer and may provide one or more of the following advantages: prevent (or minimize) contact between the drug and alkaline buffer during processing or storage; prevent (or minimize) static; prevent (or minimize) particle attrition; avoid potential instability that may result from the proximity of the weakly basic drug and the alkaline buffer during drug laying or storage (e.g., formation of an addition compound between the drug and buffer); and insure that the alkaline buffer and the weakly basic drug do not come into direct contact until the dosage form comes into contact with a dissolution medium or body fluid following oral ingestion. In one embodiment, the sealant layer comprises a hydrophilic polymer. Non-limiting examples of suitable hydrophilic polymers include hydrophilic hydroxypropylcellulose (e.g., Klucel® LF), hydroxypropyl methylcellulose or hypromellose (e.g., Opadry® Clear or Pharmacoat™ 603), vinylpyrrolidone-vinylacetate copolymer (e.g., Kollidon® VA 64 from BASF), and low-viscosity ethylcellulose (e.g., viscosity of 10 cps or less a 5% solution in 80/20 toluene/ alcohol at 25°C as measured using an Ubbelohde viscometer). The sealant layer can constitute from about 1% to about 20% of the weight of the drug-containing, sealant-coated core, for example about 1%, about 2%, about 3%, about 4%, about 5%, about 7%, about 10%, about 12%, about 15%, about 17%, or about 20%, inclusive of all ranges and subranges therebetween.

In some embodiments, the microparticles of the present invention comprise a controlled-release coating comprising a water-insoluble polymer disposed on the alkaline buffer layer. In the embodiments, the controlled-release coating comprises the water-insoluble polymer in the absence of a water-soluble or enteric polymer and the controlled-release coating sustains release of the drug over from about 8 hours to about 20 hours, when tested in the two-stage dissolution method (700 mL of 0.1N HCl (hydrochloric acid) for the first 2 hours and thereafter in 900 mL at pH 6.8 obtained by adding 200 mL of a pH modifier), suitable for a once- or twice-daily dosing regimen.

Non-limiting examples of suitable water-insoluble polymers include ethylcellulose, cellulose acetate, cellulose acetate butyrate, polyvinyl acetate, neutral methacrylic acid-methylmethacrylate copolymers, and mixtures thereof. In one embodiment, the water-insoluble polymer comprises ethylcellulose. In another embodiment, the water-insoluble polymer comprises ethylcellulose with a mean viscosity of 10 cps in a 5% solution in 80/20 toluene/alcohol measured at 25° C on an Ubbelohde viscometer. The water-insoluble polymer of the sustained-release coating provides a weight gain from about 3% to about 30%, including about 3%, about 5%, about 7%, about 10%, about 12%, about 15%, about 17%, about 20%, about 22%, about 25%, about 27%, about 30%, about 35%, and about 40%, inclusive of all ranges and subranges therebetween. In one example, the sustained-release microparticle may have a sustained-release coating of a plasticized water-insoluble polymer, such as ethylcellulose (EC-10), at about 5-50% by weight to sustain the drug release over about 4-20 hours.

In one embodiment, the water-insoluble polymer of the controlled-release coating further comprises a plasticizer. Non-limiting examples of suitable plasticizers include triacetin, tributyl citrate, triethyl citrate, acetyl tri-n-butyl citrate, diethyl phthalate, castor oil, dibutyl sebacate, monoacetylated and diacetylated glycerides (e.g., Myvacet® 9-45), and mixtures thereof. When used in an embodiment of the present invention, the plasticizer may constitute from about 3% to about 30% by weight of the water-insoluble polymer. In another embodiment, the plasticizer constitutes from 10% to about 25% by weight of the water-insoluble polymer. In still other embodiments, the amount of plasticizer relative to the weight of the water-insoluble polymer is about 3%, about 5%, about 7%, about 10%, about 12%, about 15%, about 17%, about 20%, about 22%, about 25%, about 27%, and about 30%, inclusive of all ranges and subranges therebetween. One of ordinary skill in the art will recognize that the type(s) and amount(s) of plasticizer(s) can be selected based on the polymer or polymers and nature of the coating system (e.g., aqueous or solvent-based, solution or dispersion-based and the total solids). In one embodiment, if a plasticizer is used in the controlled-release coating, the plasticizer is free of phthalates.

In one embodiment of the present invention, in each coating layer where a plasticizer is present, the plasticizer(s) is free of phthalates.

Also disclosed are examples wherein, the controlled-release coating disposed on the alkaline buffer layer comprises a water-insoluble polymer in combination with a water-soluble polymer and provides sustained release of the drug. In one example, the ratio of the water-insoluble polymer to the water-soluble polymer ranges from about 95/5 to about 50/50, including the range of about 90/10 to about 60/40. In another example, the water-insoluble and water-soluble polymers in combination constitute from about 3% to about 50% by weight of the coated core, including the ranges from about 10% to about 50%, about 3% to about 30%, and from about 5% to about 30%. In other examples, the amount of water-insoluble and water-soluble polymers in combination constitute about 3%, about 5%, about 7%, about 10%, about 12%, about 15%, about 17%, about 20%, about 22%, about 25%, about 27%, about 30%, about 35%, about 40%, about 45%, and about 50% of the weight of the immediate release core, inclusive of all ranges and subranges therebetween.

The water-soluble polymers used in accordance with certain examples of the present disclosure encompass water-soluble polymers. Non-limiting examples of suitable water-soluble polymers include polyvinylpyrrolidone (e.g., Povidone K-25), polyethylene glycol (e.g., PEG 400), hydroxypropyl methylcellulose, and hydroxypropylcellulose. In one example, the sustained-release coating provides a drug release sustained over about 12 to about 16 hours when tested in the two-stage dissolution method (700 mL of 0.1N HCl (hydrochloric acid) for the first 2 hours and thereafter in 900 mL at pH 6.8 obtained by adding 200 mL of a pH modifier), suitable for a once- or twice-daily dosing regimen.

In another embodiment, the controlled-release coating comprises a water-insoluble polymer in combination with a gastrosoluble pore-former and provides sustained release of the drug. An example of a gastrosoluble pore-former is calcium carbonate. Other suitable gastrosoluble pore-formers include sodium chloride, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, etc.

Also disclosed are examples wherein, the controlled-release coating comprises a water-insoluble polymer in combination with an enteric polymer and provides a delayed or a timed, pulsatile release (TPR) of the drug. This type of controlled-release coating (i.e., the combination of water-insoluble and enteric polymers) may be referred to herein as a "lag-time" coating, and the microparticles coated the lag-time coating may be referred to herein as TPR microparticles. The term "lag-time" refers to a time period after oral administration of the drug-containing particle or after exposure to the 2-stage dissolution media or simulated body fluid(s), wherein less than about 10% of the drug is released from the drug-containing particle. In one example, the term "lag-time" refers to a time period wherein substantially none of the drug is released from the particle or after exposure to the 2-stage dissolution media or simulated body fluid(s). In one example, the lag-time coating is deposited directly onto the alkaline buffer layer. In another example, the lag-time coating is deposited directly onto one or more layers (e.g., a sealant layer) coated onto the alkaline buffer layer. In some examples, the ratio of the water-insoluble polymer to enteric polymer ranges from about 10:1 to about 1:4, including the ranges of from about 9:1 to about 1:3 and from about 3:1 to about 1:1. In other examples, the water-insoluble and enteric polymers in combination constitute from about 5% to about 60% by weight of the immediate release core, including the ranges of from about 10% to about 60%, and from about 10% to about 50%. Non-limiting examples of suitable enteric polymers include cellulose acetate phthalate, hydroxypropyl methylcellulose phthalate, hydroxypropyl methylcellulose acetate succinate, polyvinyl acetate phthalate, pH-sensitive methacrylic acid-methamethacrylate copolymers, shellac, and mixtures thereof. (The term "pH sensitive" refers to polymers which pH dependent solubility.) These enteric polymers may be used as a dry powder or an aqueous dispersion. Some commercially available materials that may be used are methacrylic acid copolymers sold under the trademark Eudragit (L100, S100, L30D) manufactured by Rohm Pharma, Cellacefate (cellulose acetate phthalate) from Eastman Chemical Co., Aquateric (cellulose acetate phthalate aqueous dispersion) from FMC Corp., and Aqoat (hydroxypropyl methylcellulose acetate succinate aqueous dispersion) from Shin Etsu K. K. In one example, the TPR-coating comprises ethylcellulose (e.g., EC-10) as the water-insoluble polymer and hypromellose phthalate (e.g., HP-55) as the enteric polymer.

In one embodiment, the TPR microparticles may provide a lag time of from about 1 hour to about 10 hours, including from about 2 hours to about 7 hours, from about 2 hours to about 4 hours ("shorter lag time"), and from about 7 hours to about 8 hours ("longer lag time"). In another embodiment, the TPR microparticles release the drug over a period of about 4 hours to about 16 hours in the gastrointestinal tract after a lag time of about 1 hour to about 10 hours following oral administration.

In another embodiment, the microparticles contain an outer, lag-time coating disposed on the controlled-release coating. This type of embodiment begins to release drug in the higher pH of the intestine, followed by sustained-release of the drug.

The drug release profiles of SR and TPR microparticles may be determined by dissolution testing in a USP Apparatus 1 or 2 using a two-stage dissolution medium (first 2 hours in 700 mL of 0.1N HCl at 37°C followed by dissolution testing at pH 6.8 obtained by the addition of 200 mL of a pH modifier). Drug release with time can be determined using various methods, for example by HPLC on samples pulled at selected time points.

The SR or TPR coating contributes to the control of drug dissolution at the drug interface and hence drug release from the microparticles. The achievable lag time or sustained-release time depends on the composition and thickness of the sustained-release coating, and/or the composition and thickness of the lag-time coating. Specific factors that can affect achieving optimal twice- or once-daily dosage forms include, but are not limited to, the therapeutic agent's pKa (and its solubility, i.e., the drug being freely soluble under acidic and neutral pH conditions, but poorly soluble at or above pH 8.0), elimination half-life, and solubility reduction in the micro-alkaline pH environment created by the alkaline buffer.

In another embodiment, the microparticles contain a compressible coating disposed on the controlled-release coating (or disposed on the outer-most coating, if the controlled-release coating is further coated with a TPR coating). The compressible coating comprises a hydrophilic polymer. In one embodiment, the hydrophilic polymer is selected from the group consisting of hydroxypropylcellulose, poly(vinyl acetate-vinyl pyrrolidone), polyvinyl acetate, and plasticized low-viscosity ethylcellulose latex dispersion. This coating may be applied, for example, by fluid-bed coating with a plasticized aqueous dispersion of ethylcellulose. Its purpose is to maintain membrane integrity during compression with rapidly-dispersing microgranules.

The microparticle core comprises a weakly basic drug. In some embodiments, the core can take the form of an inert bead, a microgranule, or a drug crystal. In one embodiment, the core comprises an inert bead, coated with a drug layer comprising a weakly basic drug. The inert bead can comprise sugar, microcrystalline cellulose, mannitol-microcrystalline cellulose, silicon dioxide, etc. The core has an average particle size of not more than 400 µm, or, in another embodiment, not more than 350 µm. In one embodiment, the drug layer comprises a polymeric binder. The polymeric binder can be selected from the group consisting of hydroxypropylcellulose, povidone, methylcellulose, hydroxypropyl methylcellulose, carboxyalkylcellulose, polyethylene oxide, starch (e.g., corn starch and gelatinized corn starch), and a polysaccharide. The ratio of the drug to the polymeric binder can range from about 85:15 to about 100:0 (no binder).

The pharmaceutical compositions described herein can further comprise rapidly disintegrating granules comprising a saccharide and/or a sugar alcohol in combination with a disintegrant. The disintegrant can be selected from the group consisting of crospovidone, sodium starch glycolate, crosslinked sodium carboxymethylcellulose, and low-substituted hydroxypropylcellulose. The saccharide and/or sugar alcohol may be selected from the group consisting of lactose, sucralose, sucrose, maltose, mannitol, sorbitol, xylitol, and maltitol. The ratio of the disintegrant to the saccharide and/or sugar alcohol in the rapidly dispersing microgranules ranges from about 1/99 to about 10/90, and in some embodiments is about 5/95 (by weight). In some embodiments, the disintegrant or the saccharide and/or sugar alcohol, or both, can be present in the form of microparticles having an average particle size of about 30 µm or less. The ratio of the drug-containing microparticles to the rapidly disintegrating granules can range from about 1:6 to about 1:2.

In another embodiment, the present invention relates to pharmaceutical dosage forms comprising the microparticles described herein. The pharmaceutical dosage forms include orally disintegrating tablets (ODTs), conventional tablets, and capsules (e.g., hard-gelatin or HPMC, polysaccharide capsules). When the pharmaceutical dosage form takes the form of an ODT, the ODT substantially disintegrates within about 60 seconds after contact with saliva in the oral cavity or with simulated saliva fluid. In another embodiment, the ODT substantially disintegrates within about 30 seconds. Disintegration is tested according to USP 701 Disintegration Test. In one embodiment, the ODT comprises a therapeutically effective amount of a weakly basic drug, wherein after administration the ODT substantially disintegrates in the oral cavity of a patient forming a smooth, easy-to-swallow suspension having no gritty mouthfeel or aftertaste and provides a target PK profile (i.e., plasma concentration vs. time plot) of said the weakly basic drug suitable for a once- or twice-daily dosing regimen.

When the pharmaceutical dosage form is a tablet, it preferably has a friability of less than about 1%. When the pharmaceutical dosage form is an ODT, the tablet may also include pharmaceutically acceptable excipients suitable for use in disintegrating tablet formulations such as compressible diluents, fillers, coloring agents, and optionally a lubricant.

In some embodiments, the ODT weighs not more than about 2000 mg; for example, 2000 mg or less; 1500 mg or less; 1000 mg or less; 500 mg or less. In another embodiment, the ODT weighs not more than about 1600 mg. In another embodiment, the ODT weighs not more than about 800 mg. In another embodiment, the ODT weighs not more than 500 mg.

The ODTs comprise one or more populations of SR and/or one or more populations of TPR microparticles described herein, or mixtures thereof, combined with rapidly disintegrating microparticles. The ODTs may further comprise IR particles. For example, the pharmaceutical dosage form may comprise: SR microparticles in combination with rapidly disintegrating granules; TPR microparticles in combination with rapidly disintegrating granules; IR microparticles, SR microparticles, and rapidly dispersing granules; IR microparticles, TPR microparticles, and rapidly dispersing granules; or IR microparticles, SR microparticles, and one or more populations of TPR microparticles which may have the same or different lag times (e.g., short lag-time TPR microparticles and long lag-time TPR microparticles), combined with rapidly dispersing granules. These different combinations of microparticles can achieve different desired drug-release profiles. For example, a once-daily dosage form of an active with an elimination half-life of about 7 hours may contain a mixture of an IR bead population which provides an immediate-release pulse, a second SR bead or TPR bead population with a shorter lag time (about 2-4 hours), which provides a rapid sustained-release profile, and a third TPR bead population with a longer lag time (about 7-8 hours), which allows typically a delayed, sustained-release profile over about 8-12 hours, to maintain acceptable plasma concentrations at 12-24 hours.

When IR particles are present in the pharmaceutical dosage form, the ratio of IR particles to SR and/or TPR particles ranges from about 0:100 (no IR particles) to about 50:50. The IR particles may be taste-masked by applying a taste-masking layer that substantially masks the taste of the drug contained in the particle. These taste-masked IR particles release not more than about 10% in 3 minutes (the longest typical residence time anticipated for the ODT in the buccal cavity) when dissolution tested in simulated saliva fluid (pH ∼ 6.8) while releasing not less than about 75% of the dose in about 60 minutes when dissolution tested in 0.1N HCl.

The IR particles comprise a drug-containing core optionally coated with water-insoluble polymer (e.g., ethylcellulose), providing a taste-masking layer. The coating of water-insoluble polymer may comprise a plasticizer. It can further comprise a gastrosoluble pore-former (e.g., calcium carbonate), for example in accordance with the disclosure in the co-pending US Patent Application Ser. No. 11/213,266 filed Aug. 26, 2005 (Publication No. U.S. 2006/0105038 published May 18, 2006) or by fluid-bed coating with a water-insoluble polymer (e.g., ethylcellulose with a mean viscosity of 10 cps) alone or in combination with a gastrosoluble polymer (e.g., Eudragit E100 or EPO), for example in accordance with the disclosure in the co-pending US Patent Application Ser. No. 11/248,596 filed Oct. 12, 2005 (Publication No. U.S. 2006/0078614 published Apr. 13, 2006) or a gastrosoluble pore-former (e.g., calcium carbonate), for example in accordance with the disclosure in the co-pending US Patent Application Ser. No. 11/256,653 filed Oct. 21, 2005 (Publication No. U.S. 2006/0105039 published May 18, 2006).

The ODTs described herein can have one or more of the following advantages: (i) disintegrates on contact with saliva in the oral cavity in about 60 seconds, forming a smooth, easy-to-swallow suspension comprising taste-masked and/and drug-containing particles; (ii) disintegrates within about 30 seconds when tested by the <USP 701> Disintegration Test; (iii) taste-masked IR particles, if present, provide rapid, substantially complete release of the dose upon entry into the stomach (e.g., typically greater than about 75% in about 60 minutes); and/or (iv) SR and/or TPR particles provide sustained and/or delayed release of the drug in the gastrointestinal tract.

In an embodiment of the second aspect, the present invention relates to methods of preparing a pharmaceutical composition of the microparticles described herein. In one embodiment, the method comprises: (a) preparing a core comprising a weakly basic drug; (b) coating the drug-containing core of step (a) with a sealant layer; (c) coating the sealant-layered core of step (b) with a layer comprising an alkaline buffer; and (d) coating the alkaline-buffer layered core of step (c) with a controlled-release layer to provide microparticles. The step of preparing the core may be accomplished by any of the methods known in the art; for example, layering an inert bead (e.g., sugar, microcrystalline cellulose, mannitol-microcrystalline cellulose, silicon dioxide, etc.) with a solution comprising the drug and optionally a polymeric binder (e.g., by fluid-bed or pan coating); granulating the drug with an appropriate diluent (e.g., microcrystalline cellulose and/or lactose); extruding and spheronizing the drug mixture; compressing the drug into mini-tablets of about 1-2 mm in diameter; or simply obtaining drug crystals of the desired particle size (e.g., about 50-500 µm, including 100-400 µm).

In one embodiment, the method is used to prepare a microparticle with a sustained-release coating. In this embodiment, the controlled-release coating of step (d) comprises a water-insoluble polymer for a weight gain of from about 3% to about 30% to give a SR microparticle. In a disclosed example, the method is used to prepare microparticles with a timed, pulsatile release (TPR) coating. In this example, the controlled-release coating of step (d) comprises a water-insoluble polymer and an enteric polymer for a weight gain of from about 10% to about 60% to give a TPR microparticle. In another embodiment, the method is used to prepare microparticles with a sustained-release coating underlying an outer timed-pulsatile release coating. In this embodiment, the controlled-release coating of step (d) comprises a water-insoluble polymer for a weight gain of from about 3% to about 30% to give a sustained-release microparticle. This sustained-release microparticle is further coated with a layer comprising a water-insoluble polymer and an enteric polymer to give a SR/TPR microparticle.

The present disclosure relates to a method of preparing a pharmaceutical dosage form comprising: mixing the microparticles described herein with rapidly dispersing granules comprising a saccharide and/or sugar alcohol in combination with a disintegrant; and compressing the resulting mixture into a tablet to provide an ODT. In still another example, the pharmaceutical dosage form may be prepared by filling a hard-gelatin capsule with the microparticles described herein.

In one example, the method comprises the steps of:
a) preparing weakly basic drug particles (crystals, microgranules, beads, or pellets with an average particle size of 50-500 µm, particularly of 100-400 µm), more particularly of 100-350 µm) and applying a protective seal-coat onto the drug-layered beads to produce IR beads;
b) applying an alkaline buffer layer onto the IR beads from a solution of a polymeric binder if necessary and applying a protective seal-coat onto the buffer layer;
c) applying a sustained-release coating comprising a water-insoluble polymer or a water-insoluble polymer in combination with a water-soluble polymer for a weight gain of from about 3% to 30% to produce SR particles; and/or
d) applying a lag-time coating onto SR particles or alkaline buffer layered particles a combination of water-insoluble and enteric polymers at a weight ratio of from about 10:1 to 1:4 for a weight gain of from about 10% to 60% by weight of the coated bead to produce TPR particles;
e) optionally applying a hydrophilic polymeric layer on the SR layer or the TPR layer; and
f) filling appropriate amounts of SR or TPR particles with or without IR particles into hard-gelatin capsules; or compressing them into conventional or orally disintegrating tablets (ODTs) after blending with pharmaceutically acceptable excipients and one or more bead populations (e.g., a combination of IR beads, SR beads and/or TPR beads at a desired ratio).

It is to possible to prepare drug layered pellets using Granurex by controlled spheroinization and create an alkaline buffer layer disposed over seal-coated IR pellets in the same Granurex and thereafter apply controlled-release coating in fluid-bed equipment to produce SR, ER or TPR beads.

### EXAMPLES

### Example 1:

### Deconvoluted In Vitro Drug Release Profiles for Weakly Basic Drug:

A pharmacokinetic evaluation can be undertaken to identify a set of theoretical *in vitro* drug release profiles that would allow for the once or twice daily dosage form of weakly basic drugs. Using human plasma concentration-time data upon oral single dose administration or at steady state and/or upon an intravenous (IV) profile (if available), the one or two compartmental pharmacokinetic (PK) model (e.g., a two-component model is shown in schematic below)). Both oral (PO) and IV data can be fitted simultaneously to the PK model. Using WinNonlin Software, PK parameter estimates and predictions of PO and/or IV data are performed to generate equations for simulated profiles. Formulations are developed with *in vitro* drug-release profiles that mimic the simulated, i.e., deconvoluted *in vitro* profiles or encompass the target profile window. The formulations are then tested in PK studies in adult healthy subjects.

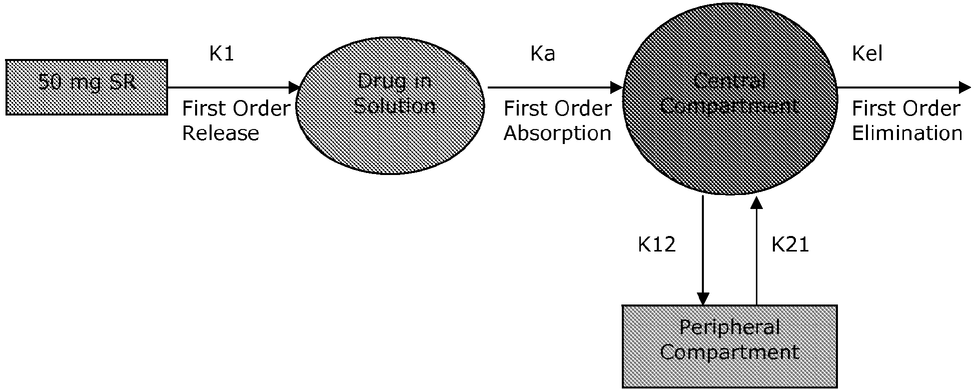

### Example 2

### 2.A IR Beads Containing Weakly Basic Drug:

A binder polymer is slowly added to a solvent system (e.g., water, acetone, ethanol or a mixture thereof) to prepare a binder solution. The weakly basic drug is slowly added to a solvent system until dissolved. The binder solution is then added to the drug solution, followed by mixing. Alternately, the binder and the drug are sequentially added to dissolve. A fluidized bed coating apparatus, e.g., a Glatt GPCG 3 (e.g., equipped with a 7" bottom spray Wurster 7 13/16"column, 'C" bottom air distribution plate covered with a 200 mesh product retention screen) is charged with (e.g., 60-80 mesh) sugar spheres, which are then sprayed with the binder/drug solution. The coated sugar spheres are then dried to drive off residual solvents (including moisture), and can be sieved (e.g., through 35 and 80 mesh screens) to discard oversized particles and fines.

### 2.B Disodium Phosphate Anhydrous (DPA) Buffer Layering:

Anhydrous disodium phosphate is added to purified water under stirring until dissolved. A fluidized bed coating apparatus, e.g., a Glatt GPCG 3 (e.g., equipped with a 6" bottom spray Wurster 8" column 13 and "C" distribution plate covered with a 200 mesh product retention screen and 1.0 mm port size nozzle) is charged with IR beads (e.g., from Example 2.A). The buffer solution is sprayed onto the IR beads. After optionally rinsing the buffer coated beads with a solvent, a seal coat of about 2% by weight is applied. The dried IR beads can be sieved (e.g., using 35 and 80 mesh sieves) to discard oversized beads and fines.

### 2.C SR Beads Containing Weakly Basic Drug:

The buffer-coated beads from Example 2.B are coated in a fluidized bed coating apparatus with a SR coating of an optionally plasticized (e.g., triethylcitrate at 10% w/w of ethylcellulose) water-insoluble polymer (e.g., ethyl cellulose). A compressible coating solution (e.g., hydroxypropylcellulose such as Klucel® LF) dissolved in a solvent is sprayed onto the buffer coated beads for a weight gain of about 2% by weight. The resulting SR beads can be dried to drive off residual solvents.

### 2.D Rapidly Dispersing Microgranules:

The rapidly dispersing microgranules are prepared following the procedure disclosed in the co-pending US Patent Application No. 10/827,106 (published as US Patent Application Publication No. U.S. 2005/0232988 on October 20, 2005). D-mannitol with an average particle size of approximately 20 µm or less (e.g., Pearlitol 25 from Roquette, France) are blended with 8 kg of crosslinked povidone (e.g., Crospovidone XL-10 from ISP) in a high shear granulator (GMX 600 from Vector) and granulated with purified water and wet-milled using Comil from Quadro and tray-dried to obtain a loss on drying (LOD) of less than about 1%. The dried granules are sieved, and oversized material is milled to produce rapidly dispersing microgranules with an average particle size in the range of approximately 175-300 µm.

### 2.E Controlled-Release ODT Containing SR Beads:

Rapidly dispersing microgranules (∼1200 g) are blended with SR beads of weakly basic drug (∼850 g) and other pharmaceutical acceptable ingredients, such as flavor (∼25 g), sweetener (e.g., sucralose, ∼10 g), additional crospovidone (∼125 g), and microcrystalline cellulose (e.g., Avicel PH101, ∼250 g) at a ratio of rapidly dispersing microgranules to SR beads of about 3:2 in a twin shell V-blender for a sufficient time to obtain a homogeneously distributed blend for compression. ODTs comprising 50 mg of weakly basic drug as SR Beads are compressed using a production scale tablet press equipped with an external lubrication system at the following conditions: - tooling: 15 mm round, flat face, radius edge; compression force: 16 kN; mean weight: 1000 mg; mean hardness: 46 N; and friability: 0.28%. The resulting ODT (50 mg dose) thus produced rapidly disintegrates in the oral cavity, creating a smooth, easy-to-swallow suspension comprising coated beads and provides an expected a drug-release profile suitable for a once-daily dosing regimen.

### Example 3

### 3.A IR Beads Containing Weakly Basic Drug:

A binder polymer is slowly added to a solvent system (e.g., water, acetone, ethanol or a mixture thereof) to prepare a binder solution. The weakly basic drug, clonidine, a phenylamino imidazoline derivative, is slowly added to the binder solution to dissolve while mixing. A fluidized bed coating apparatus, e.g., a Glatt GPCG 3 (e.g., equipped with a 7" bottom spray Wurster 7 13/16"column, 'C" bottom air distribution plate covered with a 200 mesh product retention screen) is charged with microcrystalline cellulose spheres ((e.g., Cellets 100 from Glatt), which are then sprayed with the binder/drug solution. The IR beads are then dried to drive off residual solvents (including moisture), and can be sieved (e.g., through 40 and 100 mesh screens) to discard oversized particles and fines.

### 3.B Disodium Phosphate Anhydrous (DPA) Buffer Layering:

Anhydrous disodium phosphate is added to purified water under stirring until dissolved. A fluidized bed coating apparatus, e.g., a Glatt GPCG 3 (e.g., equipped with a 6" bottom spray Wurster 8" column 13 and "C" distribution plate covered with a 200 mesh product retention screen and 1.0 mm port size nozzle) is charged with IR beads (e.g., from Example 3.A). The buffer solution is sprayed onto the IR beads. After optionally rinsing the buffer coated beads with a solvent, a seal coat of about 2% by weight is applied. The dried IR beads can be sieved (e.g., using 35 and 80 mesh sieves) to discard oversized beads and fines.

### 3.C TPR Layering:

The buffer-coated beads from Example 3.B are coated in a fluidized bed coating apparatus with a TPR coating comprising ethylcellulose (Ethocel Premium 10 cps), hypromellose phthalate (HP-55) and TEC (triethylcitrate) at a ratio of 55/30/15 dissolved in 90/10 acetone/water for a weight gain of 30% by weight of the coated bead. A compressible coating solution (e.g., hydroxypropylcellulose such as Klucel® LF) dissolved in a solvent is sprayed is sprayed onto the buffer coated beads for a weight gain of about 2% by weight. The resulting SR beads can be dried to drive off residual solvents.

### 3.D Controlled-Release ODT Containing TPR Beads:

Rapidly dispersing microgranules from Example 2.D are blended with TPR beads of weakly basic drug of Example 3.C and other pharmaceutical acceptable ingredients, such as flavor, sweetener (e.g., sucralose), additional crospovidone, and microcrystalline cellulose (e.g., Avicel PH101) at a ratio of rapidly dispersing microgranules to TPR beads of about 3:2 in a twin shell V-blender for a sufficient time to obtain a homogeneously distributed blend for compression. ODTs comprising 50 mg of weakly basic drug as TPR Beads are compressed using a production scale tablet press equipped with an external lubrication system: The resulting ODT (50 mg dose) thus produced rapidly disintegrates in the oral cavity, creating a smooth, easy-to-swallow suspension comprising coated beads and provides an expected a drug-release profile suitable for a once-daily dosing regimen.

### Example 4

### 4.A IR Beads Containing Weakly Basic Drug:

The drug layering solution in an appropriate solvent system is prepared by first adding the polymeric binder until dissolved, followed by the weakly basic drug. The solution is then applied onto Cellets 100 (microcrystalline cellulose spheres 100-200 µm in average particle size). The resulting IR beads are then dried to drive off residual solvents (including moisture), and sieved (e.g., through 40 and 100 mesh screens) to discard oversized particles and fines.

### 4.B Magnesium Oxide Buffer Layering:

Micronized magnesium oxide is added to a polymeric binder solution in an ethanol-based solvent system under stirring to provide a homogeneous dispersion. A fluidized bed coating apparatus, e.g., a Glatt GPCG 3, is charged with IR beads (e.g., from Example 4.A), and the magnesium oxide/polymeric binder solution is sprayed onto the IR beads. After optionally rinsing the buffer coated beads with a solvent, a seal coat of about 2% by weight is applied. The dried IR beads can be sieved to discard oversized beads and fines.

### 4.C TPR Layering:

The buffer-coated beads from Example 4.B are coated in a fluidized bed coating apparatus with an SR coating comprising ethylcellulose (EC-10) and TEC at a ratio of 90/10 dissolved in 95/5 acetone/water for a weight gain of 10% by weight of the coated bead. The SR coated beads are further coated with a TPR coating solution comprising EC-10, HP-55 and TEC at a ratio of 60/30/10, followed by a compressible coating with Klucel® LF for a weight gain of about 2% by weight. The resulting TPR beads can be dried to drive off residual solvents.

### 4.D Taste-masked IR Beads

IR beads from Example 4.A are taste masked by coating with EC-10 and Eudragit® E100, TEC, and magnesium stearate in the Glatt GPCG 3 for a weight gain of about 15% by weight.

### 4.E Controlled-Release ODT Containing IR and TPR Beads:

Rapidly dispersing microgranules from Example 2.D, TPR beads and taste masked IR beads of weakly basic drug from Example 4.D at a ratio of 2:1 are blended with other pharmaceutical acceptable ingredients, such as flavor, sweetener (e.g., sucralose), additional crospovidone, and microcrystalline cellulose (e.g., Avicel PH101). The TPR and taste-masked IR beads are combined with rapidly dispersing granules at a ratio of rapidly dispersing microgranules to coated beads of about 3:2 in a twin shell V-blender for a sufficient time to obtain a homogeneously distributed blend for compression. ODTs comprising 50 mg of weakly basic drug as IR/TPR beads are compressed using a production scale tablet press equipped with an external lubrication system: The resulting ODT (50 mg dose) thus produced rapidly disintegrates in the oral cavity, creating a smooth, easy-to-swallow suspension comprising coated beads and provides an expected a drug-release profile suitable for a once-daily dosing regimen.

### Example 5

### 5.A IR Beads Containing Propiverine HCl

Propiverine HCl (308 g) was slowly added to purified water (2054.7 g) while stirring until dissolved. The pre-heated Glatt 3 was charged with Cellets 100 (900 g) and the drug solution was sprayed at a rate of 4 mL/min with a stepwise increase to 12 mL/min and at inlet air volume of 8 CFM, product temperature of 50±2°C. Following the rinse of the spray system with 40 g water, a seal coat at 2% of Opadry Clear (6% solids in water) was then applied and the resulting IR beads were dried to drive off residual solvents (including moisture), and sieved (e.g., through 40 and 100 mesh screens) to discard oversized particles and fines.

### 5.B Dibasic Sodium Phosphate Buffer Layering

Dibasic sodium phosphate (113.9 g) was slowly added to a polymeric binder, povidone (2.3 g) aqueous solution (2278 g water) under stirring to dissolve. The pre-heated Glatt GPCG 3 was charged with IR beads (e.g., from Example 5.A; 1000 g), and the buffer solution was sprayed onto the IR beads as disclosed in Example 3.B. After optionally rinsing the spray system with 40 g water, a seal coat of about 2% by weight with Opadry Clear was applied. The dried IR beads can be sieved to discard oversized beads and fines.

### 5.C Propiverine SR Beads (30% coating)

The buffer-coated beads (900 g) from Example 5.B were coated in the pre-heated fluidized bed coating apparatus with an SR coating comprising ethylcellulose (357.4 g) and TEC (39.7) at a ratio of 90/10 dissolved in acetone (3375 g)/water (596 g) for a weight gain of 30% by weight of the coated bead. The SR coated beads were further coated with a compressible coating with Klucel® LF (26.5 g) for a weight gain of about 2% by weight. The resulting SR beads were dried to drive off residual solvents. The SR beads with a coating of 20%, 25% and 30% by weight were dissolution tested by the two-stage dissolution methodology (USP Apparatus 2 (paddles @ 50 RPM, dissolution media: 700 mL 0.1N HCl for the first 2 hours and thereafter at pH 6.8 achieved by adding 200 mL of buffer modifier at 37°C)). The dissolution data are presented in Table 2 below. It is clear from the table, the coating level requires to be significantly lowered.

**Table 2: In vitro Drug release profiles of Propiverine HCl SR Beads**

| Time (hours) | Drug Released (%) | | |
|---|---|---|---|
| | 20% Coating | 25% Coating | 30% Coating |
| 1.0 | 0.91 | 1.65 | 3.71 |
| 2.0 | 3.08 | 5.09 | 9.92 |
| 4.0 | 4.26 | 7.74 | 15.8 |
| 8.0 | 7.29 | 12.8 | 26.1 |
| 12.0 | 9.49 | 16.4 | 32.1 |
| 16.0 | 11.2 | 19.2 | 35.8 |
| 24.0 | 13.6 | 22.7 | 40.5 |

### Example 6

### 6.A IR Beads Containing Propiverine HCl

Propiverine HCl (256.5 g) was slowly added to 50/50 acetone/water (855 g each) while stirring until dissolved, and then add sodium stearyl fumarate (PRUV; 28.5 g) was added while vigorously stirring to evenly disperse. The pre-heated Glatt 3 was charged with 45-60 mesh sugar spheres (972 g) and the drug solution (continually being stirred during spraying) was sprayed at a rate of 4 mL/min with a stepwise increase to 8 mL/min and at inlet air volume of 10 CFM, product temperature of 45±2°C. Following the rinse of the spray system with 40 g acetone, a seal coat at 2% of Opadry Clear (6% solids in water) was then applied and the resulting IR beads were dried to drive off residual solvents (including moisture), and sieved (e.g., through 40 and 80 mesh screens) to discard oversized particles and fines.

### 6.B Dibasic Sodium Phosphate Buffer Layering

Dibasic sodium phosphate (113.9 g) was layered on IR beads (e.g., from Example 5.A; 1000 g) in the pre-heated Glatt GPCG 3 following the procedures as disclosed in Example 5.B. After optionally rinsing the spray system with 40 g acetone, a seal coat of about 2% by weight with Opadry Clear was applied. The dried IR beads can be sieved to discard oversized beads and fines.

### 6.C Propiverine SR Beads (10% coating)

The buffer-coated beads (850 g) from Example 6.B were coated in the pre-heated fluidized bed coating apparatus with an SR coating comprising ethylcellulose (86.9 g) and TEC (9.7 g) at a ratio of 90/10 dissolved in acetone (821 g)/water (145 g) for a weight gain of 10% by weight of the coated bead. The SR coated beads were further coated with a compressible coating with Klucel® LF (19.3 g) for a weight gain of about 2% by weight. The resulting SR beads were dried to drive off residual solvents.

### 6.D Propiverine HCl ODT CR

Rapidly dispersing microgranules (43.68 parts) from Example 2.D, propiverine HCl SR beads (34.97 parts) from Example 6.C, and the pre-blend (microcrystalline cellulose (Ceolus KG 802 + Avicel PH101, 7.5 parts each), crospovidone (5 parts), sucralose (0.35 part), peppermint flavor (1.0 part) were blended and passed through 40 mesh sieve to achieve a homogeneous blend) were blended in a V blender as disclosed in Example 4.E. ODT tablets comprising 50 mg propiverine HCl as SR beads were compressed using a production scale tablet press equipped with an external lubrication system: The resulting ODT (50 mg dose) thus produced rapidly disintegrates in the oral cavity, creating a smooth, easy-to-swallow suspension comprising coated beads and the disintegration time when tested per the USP method <701> was less than 30 seconds.

### Example 7

### 7.A Propiverine HCl Pellets by Controlled Spheronization

Povidone (PVP K-30; 111.1 g) and propiverine HCl (particle size distribution - D(0.1): 2.6 µm; D(0.5): 10.38 µm; D(0.9): 42.52 µm; 1000 g) are blended together and charged into the product bowl of Granurex GX-35 from Vector Corporation (Iowa, USA). Purified water is sprayed into the rotating material bed at a controlled rate. Optimized parameters during forming pellets - Process air temperature: ∼ 19-20°C; Product temperature: 16±2°C; Rotor speed: 425 RPM; External air supply: 150 L/min; Spray rate: 15 RPM (∼ 8 mL/min); pressure drop across slit: 1.3-11 mm in water; and during drying of pellets - Process air volume: 30 CFM; Process air temperature: ∼ 60°C; Product temperature: 35°C (to stop drying); rotor speed: 180 RPM; slit air volume: 10 CFM; processing time: 40 min. The pellets thus prepared have about 65% of the particles in the size range of 40-80 mesh.

### 7.B Taste-masked Propriverine HCl IR Pellets

Pellets (970 g) from Example 7.A are seal coated with Klucel LF (30 g) dissolved in acetone/water (7.5% solids) for a weight gain of 3%. Ethylcellulose (EC-10, Ethocel Premium 10 from Dow Chemicals; 159.1 g) is slowly added to 85/15 acetone/water (10% solids) while stirring constantly until dissolved. Triethyl citrate (TEC; 15.9 g) is slowly added until dissolved. These IR pellets are taste-masked in Glatt GPCG 3 by spraying the above solution for a weight gain of 20%.

### 7.C Magnesium Oxide Layering on Propiverine Pellets by Powder-layering

Povidone (11.9 g) is slowly added to purified water (5% solids) while stirring to dissolve. Propriverine pellets from Example 7.A (2000 g) or seal-coated propiverine pellets from Example 7.B (2000 g) are charged into the product bowl of Granurex GX-35 from Vector Corporation (Iowa, USA). The povidone solution is sprayed into the rotating material bed at a controlled rate while simultaneously the powder (229.3 g of magnesium oxide) is sprayed into the unit with a powder layer (K-Tron) at a controlled rate. Optimized parameters during powder-layering - Product temperature: 22-25°C; Rotor speed: 300 RPM; External air supply: 150-320 L/min and temperature: 100°C; pressure drop across slit: 1-2 mm of water; Solution spray rate: 3-5 mL/min (nozzle air: 20 PSI); and powder spray rate: 5 g/min (air pressure: 12.5 PSI). The pellets are coated with a seal coat by spraying the same binder solution or an Opadry Clear solution (5% solids) at a process air volume of 70 CFM and the seal coated buffer-layered pellets are dried in the unit for about 5 minutes to reduce moisture content.

The milled or micronized drug substance may be blended with flow aids such as colloidal silica or magnesium stearate. The binder up to 10% may be partially blended with the drug powder and partly dissolved in the spray fluid. Solvents (e.g., acetone, ethanol or a mixture) may be used in a solvent rated Granurex unit. An alternate binder such as Klucel LF, hypromellose may also be used. The spheronized pellets may be applied a protective seal coat with Opadry Clear, or Klucel LF in the Granurex itself or in a fluid-bed coater as disclosed in Example 6.B above.

### 7.D Propriverine HCl CR Pellets (25% TPR/10% SR coating)

As disclosed in Example 2.C, buffer coated propiverine pellets from Example 7.B (900 g) are coated in a pre-heated fluidized bed coater, GPCG 3 with an SR coating at 10% by weight comprising ethylcellulose (Ethocel Premium 10 cps; 128.6 g) plasticized with triethylcitrate (14.3 g), and further coated with a TPR coating comprising ethylcellulose (214.3 g), hypromellose phthalate (HP-55; 107.2 g) and TEC (triethylcitrate, 35.7 g) at a ratio of 60/30/10 dissolved in 85/15 acetone/water for a weight gain of 25% by weight of the coated pellet. A compressible coating solution of Klucel® LF (7.5% solids) is sprayed onto the TPR coated pellets for a weight gain of about 2% by weight. The resulting CR pellets are dried in the unit for 5 min to drive off residual solvents.

### 7.E Propriverine HCl ODT CR, 200 mg

Rapidly dispersing microgranules (57.2 parts) from Example 2.D, CR pellets (15.6 parts) from Example 6.C and taste masked IR pellets (12.8 parts) from Example 6.B are blended with a pre-blend comprising other pharmaceutical acceptable ingredients, such as flavor (1 part), sweetener (e.g., sucralose; 0.35 part), additional crospovidone (5 parts), and microcrystalline cellulose (e.g., Avicel PH101; 10 parts) and compressed into 200 mg ODT CR tablets weighing about 1250 mg using a production scale tablet press equipped with an external lubrication system: The resulting ODT (100 mg dose) thus produced would rapidly disintegrate in the oral cavity, creating a smooth, easy-to-swallow suspension comprising coated beads and provide an expected a drug-release profile suitable for a once-daily dosing regimen.

The skilled artisan will recognize that the above procedures and compositions can be suitably modified to provide the appropriate dose of weakly basic drug.

## Claims

1. A pharmaceutical composition comprising a plurality of controlled-release particles, wherein at least one population of said particles comprises:
(a) a core comprising a weakly basic drug, or a pharmaceutically acceptable salt, solvate, and/or ester thereof;
(b) an alkaline-buffer layer deposited over the core, comprising an alkaline buffer, wherein the alkaline-buffer layer may or may not be in physical contact with the core; and
(c) a controlled-release coating deposited over the alkaline-buffer layer, wherein the controlled-release coating may or may not be in physical contact with the alkaline-buffer layer,
wherein the controlled-release coating comprises a water-insoluble polymer in the absence of a water-soluble or enteric polymer, and sustains drug release over from 8 hours to 20 hours,
wherein the release is determined in a two-stage dissolution method (700 mL of 0.1N hydrochloric acid for the first 2 hours and thereafter in 900 mL at pH 6.8 obtained by adding 200 mL of a pH modifier).

2. The pharmaceutical composition of claim 1,
wherein said weakly basic drug is selected from the group consisting of analgesics, anticonvulsants, anti-cholinergic, antidiabetic agents, anti-infective agents, antineoplastics, anti-Parkinsonian agents, antirheumatic agents, cardiovascular agents, central nervous system stimulants, dopamine receptor agonists, anti-emetics, gastrointestinal agents, psychotherapeutic agents, opioid agonists, opioid antagonists, anti-epileptic drugs, histamine H₂ antagonists, anti-asthmatic agents, and skeletal muscle relaxants,
or wherein said weakly basic drug is selected from the group consisting of butyrophenone derivatives containing a nitrogen moiety, phenylamino imidazoline, dihydroxyphenyl isopropylamino ethane, phenoxy butylamino propanol, phenoxy amino propane, amino ethyl oxazolo azepine, propiverine, a pharmaceutically acceptable salt, solvate, polymorph, ester thereof, and mixtures thereof.

3. The pharmaceutical composition of claim 1, further comprising a compressible coating deposited on said controlled-release coating, wherein said compressible coating may or may not be in physical contact with the controlled-release coating.

4. The pharmaceutical composition of claim 1, wherein said water-insoluble polymer is selected from the group consisting of ethylcellulose, cellulose acetate, cellulose acetate butyrate, polyvinyl acetate, neutral methacrylic acid-methylmethacrylate copolymers, and mixtures thereof or wherein the water-insoluble polymer comprises ethylcellulose.

5. The pharmaceutical composition of claim 1, where said controlled-release coating further comprises a plasticizer.

6. The pharmaceutical composition of claim 1, wherein the ratio of said alkaline buffer to said weakly basic drug ranges from about 5:1 to about 1:5.

7. The pharmaceutical composition of claim 1, further comprising an outer, lag-time coating deposited over said controlled-release coating, wherein said lag-time coating may or may not be in physical contact with the controlled release coating,
wherein the outer, lag-time coating optionally comprises a water-insoluble polymer in combination with an enteric polymer at a ratio of said water-insoluble polymer to said enteric polymer ranges from about 9:1 to about 1:3, or
wherein optionally said water-insoluble polymer is ethylcellulose and said enteric polymer is hydroxypropyl methylcellulose phthalate.

8. The pharmaceutical composition of claim 1, wherein the core comprises an inert bead coated with a drug layer comprising the weakly basic drug.

9. The pharmaceutical composition of claim 1, wherein said composition further comprises rapidly disintegrating granules comprising a saccharide and/or sugar alcohol in combination with a disintegrant, wherein:
(a) said disintegrant is selected from the group consisting of crospovidone, sodium starch glycolate, crosslinked sodium carboxymethyl cellulose, and low-substituted hydroxypropylcellulose;
said saccharide and/or sugar alcohol is selected from the group consisting of sucralose, lactose, sucrose, maltose, mannitol, sorbitol, xylitol, maltitol, and mixtures thereof; and
the ratio of said disintegrant to said saccharide and/or sugar alcohol ranges from about 10:90 to about 1:99, or
(b) said disintegrant and said sugar alcohol and/or said saccharide are each present in the form of microparticles having an average particle size of about 30 µm or less, or
(c) the ratio of said controlled-release particles to said rapidly disintegrating granules ranges from about 1:6 to about 1:2.

10. A pharmaceutical dosage form comprising any one of the compositions of claims 1 to 9.

11. The pharmaceutical dosage form of claim 10, wherein said composition comprises a plurality of controlled-release particles and rapidly disintegrating granules, wherein at least one population of said controlled-release particles comprises:
(a) a core comprising a weakly basic drug, or a pharmaceutically acceptable salt, solvate, and/or ester thereof;
(b) an alkaline-buffer layer deposited over the core, comprising an alkaline buffer, wherein the alkaline-buffer layer may or may not be in physical contact with the core;
(c) a controlled-release coating deposited over the alkaline-buffer layer, wherein the controlled-release coating may or may not be in physical contact with the alkaline-buffer layer;
said rapidly disintegrating granules comprise a saccharide and/or sugar alcohol in combination with a disintegrant;
wherein said controlled-release coating comprises a water-insoluble polymer in the absence of a water-soluble or enteric polymer , and sustains drug release over from 8 hours to 20 hours,
wherein the release is determined in a two-stage dissolution method (700 mL of 0.1N hydrochloric acid for the first 2 hours and thereafter in 900 mL at pH 6.8 obtained by adding 200 mL of a pH modifier) and;
whereby said pharmaceutical dosage form is an orally disintegrating tablet which substantially disintegrates within about 60 seconds after contact with saliva in the oral cavity or with simulated saliva fluid.

12. The pharmaceutical dosage form of claim 10, further comprising immediate-release particles, wherein each immediate-release particle comprises a core comprising the weakly basic drug.

13. The pharmaceutical dosage form of claim 12, wherein the ratio of said immediate release particles to said controlled-release particles ranges from about 0:100 to about 50:50, or wherein said immediate-release particles release at least about 85% of the drug contained within said immediate-release particle within 15 minutes when tested for dissolution in USP Apparatus 1 (baskets at 100 rpm) or Apparatus 2 (paddles at 50 rpm) in 900 mL of 0.1N HCl at 37°C.

14. The pharmaceutical dosage form of claim 10, further comprising:
a second population of controlled-release particles, wherein each controlled-release particle of the second population comprises:
(a) a second core comprising the weakly basic drug;
(b) a second alkaline-buffer layer deposited over the second core, comprising an alkaline buffer, wherein the second alkaline-buffer layer may or may not be in physical contact with the second core; and
(c) a second controlled-release coating deposited over the second alkaline-buffer layer, wherein the second controlled-release coating may or may not be in physical contact with the second alkaline-buffer layer, wherein the second controlled-release coating comprises a water-insoluble polymer and optionally a water-soluble or enteric polymer.

15. A method of preparing a pharmaceutical composition comprising a plurality of controlled-release particles, comprising:
(a) preparing a core comprising a weakly basic drug;
(b) coating the drug-containing core of step (a) with a layer comprising an alkaline buffer; and
(c) coating the alkaline-buffer layered core of step (b) with a controlled-release coating comprising a water-insoluble polymer in the absence of a water-soluble or enteric polymer, which sustains drug release over from 8 hours to 20 hours,
wherein the release is determined in a two-stage dissolution method (700 mL of 0.1N hydrochloric acid for the first 2 hours and thereafter in 900 mL at pH 6.8 obtained by adding 200 mL of a pH modifier).

16. The composition of claim 1 for use in a method of treating a disease or medical condition, the method comprising administering to a patient in need thereof said composition.

## Patentansprüche

1. Pharmazeutische Zusammensetzung, eine Mehrzahl von Partikeln zur kontrollierten Freisetzung umfassend, wobei mindestens eine Population der Partikel Folgendes umfasst:
(a) einen Kern, ein schwach basisches Arzneimittel oder ein pharmazeutisch unbedenkliches Salz, Solvat und/oder einen Ester davon umfassend,
(b) eine alkalische Pufferschicht, die über dem Kern abgeschieden ist, einen alkalischen Puffer umfassend, wobei die alkalische Pufferschicht in physischem Kontakt mit dem Kern stehen kann oder nicht, und
(c) eine Beschichtung zur kontrollierten Freisetzung, die über der alkalischen Pufferschicht abgeschieden ist, wobei die Beschichtung zur kontrollierten Freisetzung in physischem Kontakt mit der alkalischen Pufferschicht stehen kann oder nicht,
wobei die Beschichtung zur kontrollierten Freisetzung ein wasserunlösliches Polymer in der Abwesenheit eines wasserlöslichen oder enterischen Polymers umfasst und die die Arzneimittelfreisetzung über 8 Stunden bis 20 Stunden aufrechterhält,
wobei die Freisetzung in einem zweistufigen Auflösungsverfahren bestimmt wird (700 ml 0,1N Salzsäure für die ersten 2 Stunden und danach in 900 ml bei pH-Wert 6,8, der durch Zusetzen von 200 ml eines pH-Wert-Modifizierers erhalten wird).

2. Pharmazeutische Zusammensetzung nach Anspruch 1,
wobei das schwach basische Arzneimittel ausgewählt ist aus der Gruppe bestehend aus Analgetika, Antikonvulsiva, Anticholinergikum, Antidiabetika, Antiinfektiva, Antineoplastika, Antiparkinsonmitteln, Antirheumatika, Herz-Kreislauf-Mitteln, Stimulanzien des zentralen Nervensystems, Dopamin-Rezeptor-Agonisten, Antiemetika, Magen-Darm-Mitteln, Psychotherapeutika, Opioid-Agonisten, Opioid-Antagonisten, Antiepileptika, Histamin-H2-Antagonisten, Antiasthmatika und Relaxanzien für die Skelettmuskulatur,
oder wobei das schwach basische Arzneimittel ausgewählt ist aus der Gruppe bestehend aus Butyrophenon-Derivaten, die eine Stickstoff-Gruppe enthalten, Phenylaminoimidazolin, Dihydroxyphenylisopropylaminoethan, Phenoxybutylaminopropanol, Phenoxyaminopropan, Aminoethyloxazoloazepin, Propiverine, einem pharmazeutisch unbedenklichen Salz, Solvat, Polymorph, Ester davon und Mischungen davon.

3. Pharmazeutische Zusammensetzung nach Anspruch 1, ferner eine komprimierbare Beschichtung umfassend, die auf der Beschichtung zur kontrollierten Freisetzung abgeschieden ist, wobei die komprimierbare Beschichtung in physischem Kontakt mit der Beschichtung zur kontrollierten Freisetzung stehen kann oder nicht.

4. Pharmazeutische Zusammensetzung nach Anspruch 1, wobei das wasserunlösliche Polymer ausgewählt ist aus der Gruppe bestehend aus Ethylcellulose, Celluloseacetat, Celluloseacetatbutyrat, Polyvinylacetat, neutralen Methacrylsäure-Methylmethacrylat-Copolymeren und Mischungen davon oder wobei das wasserunlösliche Polymer Ethylcellulose umfasst.

5. Pharmazeutische Zusammensetzung nach Anspruch 1, wobei die Beschichtung zur kontrollierten Freisetzung ferner einen Weichmacher umfasst.

6. Pharmazeutische Zusammensetzung nach Anspruch 1, wobei das Verhältnis des alkalischen Puffers zu dem schwach basischen Arzneimittel im Bereich von etwa 5:1 bis etwa 1:5 liegt.

7. Pharmazeutische Zusammensetzung nach Anspruch 1, ferner eine äußere Verzögerungszeit-(Lag-Time-)Beschichtung umfassend, die über der Beschichtung zur kontrollierten Freisetzung abgeschieden ist, wobei die Verzögerungszeit-Beschichtung in physischem Kontakt mit der Beschichtung zur kontrollierten Freisetzung stehen kann oder nicht,
wobei die äußere Verzögerungszeit-Beschichtung optional ein wasserunlösliches Polymer in Kombination mit einem enterischen Polymer in einem Verhältnis des wasserunlöslichen Polymers zu dem enterischen Polymer im Bereich von etwa 9:1 bis etwa 1:3 umfasst, oder
wobei optional das wasserunlösliche Polymer Ethylcellulose ist und das enterische Polymer Hydroxypropylmethylcellulosephthalat ist.

8. Pharmazeutische Zusammensetzung nach Anspruch 1, wobei der Kern ein inertes Kügelchen umfasst, das mit einer Arzneimittelschicht beschichtet ist, die das schwach basische Arzneimittel umfasst.

9. Pharmazeutische Zusammensetzung nach Anspruch 1, wobei die Zusammensetzung ferner schnell zerfallende Granulatkörner umfasst, die ein Saccharid und/oder einen Zuckeralkohol in Kombination mit einem Zerfallsmittel umfassen, wobei:
(a) das Zerfallsmittel ausgewählt ist aus der Gruppe bestehend aus Crospovidon, Natriumstärkeglycolat, vernetzter Natriumcarboxymethylcellulose und niedrig substituierter Hydroxypropylcellulose;
wobei das Saccharid und/oder der Zuckeralkohol ausgewählt ist/sind aus der Gruppe bestehend aus Sucralose, Lactose, Saccharose, Maltose, Mannitol, Sorbitol, Xylitol, Maltitol und Mischungen davon, und
das Verhältnis des Zerfallsmittels zu dem Saccharid und/oder dem Zuckeralkohol im Bereich von etwa 10:90 bis etwa 1:99 liegt, oder
(b) das Zerfallsmittel und der Zuckeralkohol und/oder das Saccharid jeweils in Form von Mikropartikeln mit einer mittleren Partikelgröße von etwa 30 µm oder weniger vorhanden sind, oder
(c) das Verhältnis der Partikel zur kontrollierten Freisetzung zu den schnell zerfallenden Granulatkörnern im Bereich von etwa 1:6 bis etwa 1:2 liegt.

10. Pharmazeutische Darreichungsform, eine der Zusammensetzungen nach den Ansprüchen 1 bis 9 umfassend.

11. Pharmazeutische Darreichungsform nach Anspruch 10, wobei die Zusammensetzung eine Mehrzahl von Partikeln zur kontrollierten Freisetzung und schnell zerfallende Granulatkörner umfasst, wobei mindestens eine Population der Partikel zur kontrollierten Freisetzung Folgendes umfasst:
(a) einen Kern, ein schwach basisches Arzneimittel oder ein pharmazeutisch unbedenkliches Salz, Solvat und/oder einen Ester davon umfassend,
(b) eine alkalische Pufferschicht, die über dem Kern abgeschieden ist, einen alkalischen Puffer umfassend, wobei die alkalische Pufferschicht in physischem Kontakt mit dem Kern stehen kann oder nicht, und
(c) eine Beschichtung zur kontrollierten Freisetzung, die über der alkalischen Pufferschicht abgeschieden ist, wobei die Beschichtung zur kontrollierten Freisetzung in physischem Kontakt mit der alkalischen Pufferschicht stehen kann oder nicht,
wobei die schnell zerfallenden Granulatkörner ein Saccharid und/oder einen Zuckeralkohol in Kombination mit einem Zerfallsmittel umfassen,
wobei die Beschichtung zur kontrollierten Freisetzung ein wasserunlösliches Polymer in der Abwesenheit eines wasserlöslichen oder enterischen Polymers umfasst und die die Arzneimittelfreisetzung über 8 Stunden bis 20 Stunden aufrechterhält,
wobei die Freisetzung in einem zweistufigen Auflösungsverfahren bestimmt wird (700 ml 0,1N Salzsäure für die ersten 2 Stunden und danach in 900 ml bei pH-Wert 6,8, der durch Zusetzen von 200 ml eines pH-Wert-Modifizierers erhalten wird), und
wodurch die pharmazeutische Darreichungsform eine oral zerfallende Tablette ist, die im Wesentlichen innerhalb von etwa 60 Sekunden nach Kontakt mit Speichel in der Mundhöhle oder mit simuliertem Speichelfluid zerfällt.

12. Pharmazeutische Darreichungsform nach Anspruch 10, ferner Partikel zur sofortigen Freisetzung umfassend, wobei jedes Partikel zur sofortigen Freisetzung einen Kern umfasst, der das schwach basische Arzneimittel umfasst.

13. Pharmazeutische Darreichungsform nach Anspruch 12, wobei das Verhältnis der Partikel zur sofortigen Freisetzung zu den Partikeln zur kontrollierten Freisetzung im Bereich von etwa 0:100 bis etwa 50:50 liegt oder wobei die Partikel zur sofortigen Freisetzung mindestens etwa 85 % des Arzneimittels, das in dem Partikel zur sofortigen Freisetzung enthalten ist, innerhalb von 15 Minuten freisetzen, wenn die Auflösung in einem USP-Apparat 1 (Körbe mit 100 U/min) oder Apparat 2 (Paddel mit 50 U/min) in 900 ml 0,1N HCl bei 37 °C geprüft wird.

14. Pharmazeutische Darreichungsform nach Anspruch 10, ferner umfassend:
eine zweite Population von Partikeln zur kontrollierten Freisetzung, wobei jedes Partikel zur kontrollierten Freisetzung der zweiten Population Folgendes umfasst:
(a) einen zweiten Kern, der das schwach basische Arzneimittel umfasst,
(b) eine zweite alkalische Pufferschicht, die über dem zweiten Kern abgeschieden ist, einen alkalischen Puffer umfassend, wobei die zweite alkalische Pufferschicht in physischem Kontakt mit dem zweiten Kern stehen kann oder nicht, und
(c) eine zweite Beschichtung zur kontrollierten Freisetzung, die über der zweiten alkalischen Pufferschicht abgeschieden ist, wobei die zweite Beschichtung zur kontrollierten Freisetzung in physischem Kontakt mit der zweiten alkalischen Pufferschicht stehen kann oder nicht, wobei die zweite Beschichtung zur kontrollierten Freisetzung ein wasserunlösliches Polymer und optional ein wasserlösliches oder enterisches Polymer umfasst.

15. Verfahren zur Herstellung einer pharmazeutischen Zusammensetzung, die eine Mehrzahl von Partikeln zur kontrollierten Freisetzung umfasst, umfassend:
(a) Herstellen eines Kerns, der ein schwach basisches Arzneimittel umfasst,
(b) Beschichten des arzneimittelhaltigen Kerns von Schritt (a) mit einer Schicht, die einen alkalischen Puffer umfasst, und
(c) Beschichten des mit einer alkalischen Pufferschicht versehenen Kerns von Schritt (b) mit einer Beschichtung zur kontrollierten Freisetzung, die ein wasserunlösliches Polymer in der Abwesenheit eines wasserlöslichen oder enterischen Polymers umfasst und die die Arzneimittelfreisetzung über 8 Stunden bis 20 Stunden aufrechterhält,
wobei die Freisetzung in einem zweistufigen Auflösungsverfahren bestimmt wird (700 ml 0,1N Salzsäure für die ersten 2 Stunden und danach in 900 ml bei pH-Wert 6,8, der durch Zusetzen von 200 ml eines pH-Wert-Modifizierers erhalten wird).

16. Zusammensetzung nach Anspruch 1 zur Verwendung in einem Verfahren zum Behandeln einer Erkrankung oder eines Leidens, wobei das Verfahren das Verabreichen der Zusammensetzung an einen Patienten, der dessen bedarf, umfasst.

## Revendications

1. Composition pharmaceutique comprenant une pluralité de particules à libération contrôlée, au moins une population desdites particules comprenant :
(a) un noyau comprenant une substance médicamenteuse faiblement basique ou un sel, un solvate et/ou un ester pharmaceutiquement acceptable de celle-ci ;
(b) une couche d'un tampon alcalin déposée sur le noyau et comprenant un tampon alcalin, la couche de tampon alcalin pouvant ou non être en contact physique avec le noyau ; et
(c) un enrobage à libération contrôlée déposé sur la couche de tampon alcalin, l'enrobage à libération contrôlée pouvant ou non être en contact physique avec la couche de tampon alcalin,
où l'enrobage à libération contrôlée comprend un polymère insoluble dans l'eau en l'absence d'un polymère hydrosoluble ou entérosoluble et assure une libération de la substance médicamenteuse sur une période qui va de 8 heures à 20 heures,
où la libération est évaluée par une méthode de dissolution à deux étapes (dans 700 ml d'acide chlorhydrique 0,1N pendant les 2 premières heures puis dans 900 ml à pH 6,8, le pH étant ajusté par l'addition de 200 ml d'un modificateur du pH).

2. Composition pharmaceutique de la revendication 1,
où ladite substance médicamenteuse faiblement basique est sélectionnée dans le groupe consistant en les suivantes : analgésiques, anticonvulsivants, agents anticholinergiques, agents antidiabétiques, agents anti-infectieux, antinéoplasiques, agents antiparkinsoniens, agents antirhumatismaux, agents cardiovasculaires, stimulants du système nerveux central, agonistes des récepteurs dopaminergiques, antiémétiques, agents gastro-intestinaux, agents psychothérapeutiques, agonistes des récepteurs opiacés, antagonistes des récepteurs opiacés, médicaments antiépileptiques, antihistaminiques des récepteurs H₂, agents antiasthmatiques et relaxants des muscles squelettiques,
ou ladite substance médicamenteuse faiblement basique est sélectionnée dans le groupe consistant en les suivantes : dérivés de la butyrophénone contenant un fragment azoté, phénylamino-imidazoline, dihydroxyphényl-isopropylamino-éthane, phénoxy-butylamino-propanol, phénoxy-amino-propane, aminoéthyl-oxazolo-azépine, propivérine et sel, solvate, polymorphe ou ester pharmaceutiquement acceptable et mélanges de celles-ci.

3. Composition pharmaceutique de la revendication 1 comprenant en outre un enrobage compressible déposé sur ledit enrobage à libération contrôlée, ledit enrobage compressible pouvant ou non être en contact physique avec l'enrobage à libération contrôlée.

4. Composition pharmaceutique de la revendication 1, où ledit polymère insoluble dans l'eau est sélectionné dans le groupe consistant en l'éthylcellulose, l'acétate de cellulose, l'acétobutyrate de cellulose, le poly(acétate de vinyle), des copolymères neutres acide méthacrylique-méthacrylate de méthyle et des mélanges de ceux-ci, le polymère insoluble dans l'eau comprenant l'éthylcellulose.

5. Composition pharmaceutique de la revendication 1, où ledit enrobage à libération contrôlée comprend en outre un plastifiant.

6. Composition pharmaceutique de la revendication 1, où le rapport entre ledit tampon alcalin et ladite substance médicamenteuse faiblement basique va de 5:1 environ à 1:5 environ.

7. Composition pharmaceutique de la revendication 1 comprenant en outre un enrobage externe à libération retardée déposé sur ledit enrobage à libération contrôlée, ledit enrobage à libération retardée pouvant ou non être en contact physique avec l'enrobage à libération contrôlée,
où l'enrobage externe à libération retardée comprend éventuellement un polymère insoluble dans l'eau en combinaison avec un polymère entérosoluble, le rapport entre ledit polymère insoluble dans l'eau et ledit polymère entérosoluble allant de 9:1 environ à 1:3 environ ou
où éventuellement ledit polymère insoluble dans l'eau est l'éthylcellulose et ledit polymère entérosoluble est le phtalate d'hydroxypropylméthylcellulose.

8. Composition pharmaceutique de la revendication 1, où le noyau comprend une bille inerte enrobée d'une couche d'une substance médicamenteuse comprenant la substance médicamenteuse faiblement basique.

9. Composition pharmaceutique de la revendication 1, où ladite composition comprend en outre des granules à désintégration rapide comprenant un saccharide et/ou un alcool de sucre en combinaison avec un désintégrant, où :
(a) ledit désintégrant est sélectionné dans le groupe consistant en la crospovidone, le glycolate d'amidon sodique, la carboxyméthylcellulose sodique réticulée et l'hydroxypropylcellulose faiblement substituée ;
ledit saccharide et/ou alcool de sucre est sélectionné dans le groupe consistant en le sucralose, le lactose, le sucrose, le maltose, le mannitol, le sorbitol, le xylitol, le maltitol et des mélanges de ceux-ci ; et
le rapport entre ledit désintégrant et ledit saccharide et/ou alcool de sucre va de 10:90 environ à 1:99 environ ou
(b) ledit désintégrant et ledit alcool de sucre et/ou ledit saccharide sont chacun présents sous la forme de microparticules ayant une dimension particulaire moyenne de 30 µm environ ou moins ou
(c) le rapport entre lesdites particules à libération contrôlée et lesdits granules à désintégration rapide va de 1:6 environ à 1:2 environ.

10. Forme galénique pharmaceutique comprenant l'une quelconque des compositions des revendications 1 à 9.

11. Forme galénique pharmaceutique de la revendication 10, où ladite composition comprend une pluralité de particules à libération contrôlée et des granules à désintégration rapide, au moins une population desdites particules à libération contrôlée comprenant :
(a) un noyau comprenant une substance médicamenteuse faiblement basique ou un sel, un solvate et/ou un ester pharmaceutiquement acceptable de celle-ci ;
(b) une couche d'un tampon alcalin déposée sur le noyau et comprenant un tampon alcalin, la couche de tampon alcalin pouvant ou non être en contact physique avec le noyau ;
(c) un enrobage à libération contrôlée déposé sur la couche de tampon alcalin, l'enrobage à libération contrôlée pouvant ou non être en contact physique avec la couche de tampon alcalin ;
lesdits granules à désintégration rapide comprenant un saccharide et/ou un alcool de sucre en combinaison avec un désintégrant ;
où ledit enrobage à libération contrôlée comprend un polymère insoluble dans l'eau en l'absence d'un polymère hydrosoluble ou entérosoluble et assure une libération de la substance médicamenteuse sur une période qui va de 8 heures à 20 heures,
où la libération est évaluée par une méthode de dissolution à deux étapes (dans 700 ml d'acide chlorhydrique 0,1N pendant les 2 premières heures puis dans 900 ml à pH 6,8, le pH étant ajusté par l'addition de 200 ml d'un modificateur du pH) et ;
où ladite forme galénique pharmaceutique est un comprimé à désintégration orale qui se désintègre essentiellement en l'espace de 60 secondes environ après contact avec la salive dans la cavité buccale ou avec un fluide simulant la salive.

12. Forme galénique pharmaceutique de la revendication 10 comprenant en outre des particules à libération immédiate, chaque particule à libération immédiate comprenant un noyau qui comprend la substance médicamenteuse faiblement basique.

13. Forme galénique pharmaceutique de la revendication 12, où le rapport entre lesdites particules à libération immédiate et lesdites particules à libération contrôlée va de 0:100 environ à 50:50 environ ou où lesdites particules à libération immédiate libèrent au moins 85 % environ de la substance médicamenteuse contenue dans ladite particule à libération immédiate en l'espace de 15 minutes au test de dissolution dans l'appareillage de la Pharmacopée des États-Unis 1 (paniers à 100 t/min) ou 2 (palettes à 50 t/min) dans 900 ml de HCl 0,1N à 37 °C.

14. Forme galénique pharmaceutique de la revendication 10 comprenant en outre :
une deuxième population de particules à libération contrôlée, chaque particule à libération contrôlée de la deuxième population comprenant :
(a) un deuxième noyau comprenant la substance médicamenteuse faiblement basique ;
(b) une deuxième couche d'un tampon alcalin déposée sur le deuxième noyau et comprenant un tampon alcalin, la deuxième couche de tampon alcalin pouvant ou non être en contact physique avec le deuxième noyau ; et
(c) un deuxième enrobage à libération contrôlée déposé sur la deuxième couche de tampon alcalin, le deuxième enrobage à libération contrôlée pouvant ou non être en contact physique avec la deuxième couche de tampon alcalin, où le deuxième enrobage à libération contrôlée comprend un polymère insoluble dans l'eau et éventuellement un polymère hydrosoluble ou entérosoluble.

15. Procédé de préparation d'une composition pharmaceutique comprenant une pluralité de particules à libération contrôlée, qui comprend :
(a) la préparation d'un noyau comprenant une substance médicamenteuse faiblement basique ;
(b) l'enrobage du noyau contenant une substance médicamenteuse de l'étape (a) avec une couche qui comprend un tampon alcalin ; et
(c) l'enrobage du noyau sur laquelle une couche de tampon alcalin a été déposée de l'étape (b) avec un enrobage à libération contrôlée qui comprend un polymère insoluble dans l'eau en l'absence d'un polymère hydrosoluble ou entérosoluble et assure une libération de la substance médicamenteuse sur une période qui va de 8 heures à 20 heures,
où la libération est évaluée par une méthode de dissolution à deux étapes (dans 700 ml d'acide chlorhydrique 0,1N pendant les 2 premières heures puis dans 900 ml à pH 6,8, le pH étant ajusté par l'addition de 200 ml d'un modificateur du pH).

16. Composition de la revendication 1 pour une utilisation dans une méthode de traitement d'une maladie ou d'une affection médicale, la méthode comprenant l'administration de ladite composition à un patient qui le requiert.
